# EUROPEAN PATENT SPECIFICATION

(11) **EP 0 783 910 B1**
(45) Date of publication and mention of the grant of the patent: **12.07.2000**
(21) Application number: 94927126.6
(22) Date of filing: 19.08.1994
(51) Int. Cl.: B01D 3/10, C10G 7/06

(54) **PROCESS FOR THE VACUUM DISTILLATION OF CRUDE PETROLEUM, AND A FACILITY FOR CARRYING OUT THE PROCESS**
VERFAHREN ZUR VAKUUMDESTILLATION VON ROHÖL UND EINE ANLAGE ZUR DURCHFÜHRUNG DES VERFAHREN
PROCEDE DE DISTILLATION SOUS VIDE DU PETROLE BRUT ET SON INSTALLATION DE MISE EN OEUVRE

(43) Date of publication of application: 16.07.1997
(73) Proprietor: Tsegelsky, Valery Grigorievich, Moscow, 109457 (RU)
(72) Inventor: TSEGELSKY, Valery Grigorievich, Moscow, 109457 (RU); ABROSIMOV, Alexandr Alexeevich, Moscow, 109429 (RU); KOCHEMASOV, Alexandr Mikhailovich, Moscow, 109429 (RU); KOCHERGIN, Ivan Alexandrovich, Moscow, 109429 (RU)
(74) Representative: Godwin, Edgar James
(86) International application number: PCT/RU94/00197
(87) International publication number: WO 96/05900

(56) References cited:
- DE-A- 2 725 534
- DE-A- 3 149 847
- SU-A- 724 149
- SU-A- 740 808
- US-A- 3 579 307
- US-A- 3 796 640
- US-A- 4 554 055
- E.P. ZAPOROZHETS et al., "Intensifikatsia Protsessoo Khimicheskoi Tekhnologu Ezhektsionnymi Struinymi Techeniyami Zhidkosti i Gaza", 1991, Khimicheskaya Promyshlennost, No. 8.

## Description

This invention relates to a method and equipment for vacuum distillation of a petroleum product, and may be used in the petroleum refining industry for fractionating of petroleum base stock in a vacuum column.

Equipment for vacuum distillation of petroleum stock is known which includes a vacuum column equipped with a side section, wherein the reduced pressure is produced using a jet blower (ejector), and wherein the active (ejecting) medium used is steam (US-A-2073446 and US-A-2140342).

However, the deficiency of the known method and equipment is in mixing of petroleum fractions with steam and, accordingly, carrying away by steam a portion of petroleum fractions, the latter leading to contaminating of steam and to reducing the equipment efficiency.

A method is known for vacuum distillation of a liquid product which comprises feeding it to a reservoir under vacuum, separating the liquid product in the reservoir into gases and vapours of readily volatile fractions and into at least one liquid fraction, pumping out from the upper reservoir volume the gases and vapours using a vacuum-creating apparatus through a condensing cooler, and discharging them from the latter (K.P. Shumski "Vacuum apparatus and equipment in chemical engineering", Moscow, Mashinostroyeniye Publishers, 1974, pages 123, 143, 153).

Equipment is known as well for vacuum distillation of petroleum stock which comprises a vacuum column with lines for feeding the petroleum stock and removing the liquid fraction, and a line for removing gases and vapours of easily volatile (upper) petroleum fractions from the top of the column, which is connected to a vacuum-creating device (ejector-type steam pump). The line between the top of the column and the vacuum-creating apparatus comprises a cooling condenser (Petroleum refiner Handbook, Ed. G.A. Lastovkin, Leningrad, Khimiya Publisher, 1989, page 74).

According to the known method and equipment, the vacuum value in the column is achieved using ejector-type steam pump, in which the ejecting (working) medium used is steam.

The steam mixes with petroleum vapors and decomposition gases, this resulting in contamination of steam condensate with the former products, and in removing by the steam condensate of the top petroleum fractions. Moreover, the ejector-type steam pump does not provide compression of hydrocarbon gases which are not condensed after the cooling condenser to a pressure necessary for feeding the said gases to, e.g., fuel collector of petroleum refinery plant. Therefore, the said gases are to be flared off if additional compressor equipment is absent.

The condensed matter from vacuum-creating device is delivered to a settling tank wherein the petroleum product is separated from aqueous condensate. Settling tanks represent obvious sources of environmental pollution.

Another shortcoming of the known method and equipment is in condensing of easily volatile fractions, before the vacuum-creating device, in the cooling condenser; this, due to pressure differential in the latter, results either in higher pressure at the top of the column leading to reduced yield of volatile (light) products, or to a power increase provided for the vacuum-creating device.

US-A-3 579 307 discloses a method for separating a liquid product into a vapor and liquid fraction used in a vacuum evaporator. The vapor phase is condensed and adsorbed in a liquid flow in an ejector which creates vacuum in the evaporator. In this method, however, the working fluid fed to the ejector is water or water mixed with a small amount of a condensate of a gaseous monomer evaporated in the evaporator. Therefore, to reduce the monomer concentration in the working fluid, which tends to increase, the working fluid has to be constantly diluted with water, which is unacceptable if an environment-friendly method is to be achieved.

The object of the invention is to create a method and equipment for vacuum distillation of a liquid petroleum product which could provide intensification of the vacuum distillation by producing a closed circuit vacuum-creating device, in order to reduce environmental pollution and to reduce power consumption, along with equipment efficiency increase.

The invention provides a method for vacuum distillation of a petroleum product: comprising feeding the petroleum product to a vacuum fractionating column in which the petroleum product is separated into a vapour-gaseous phase containing vapours of petroleum fractions, at least one liquid fraction, and a residue; feeding part of the at least one liquid fraction to an upper portion of the vacuum fractionating column; and feeding the vapour-gaseous phase to a vacuum-creating device which includes a liquid-gaseous ejector having an inlet to which a fluid working medium is delivered; characterised by: providing condensation of the vapour-gaseous phase in the vacuum-creating device to produce a mixture which consists of a gaseous phase and a liquid phase comprising a condensate of petroleum fraction vapours; separating the said mixture into the gaseous phase and the liquid phase and discharging the gaseous phase and part of the liquid phase from the vacuum-creating device; and using as a part of the fluid working medium in the ejector a condensate of petroleum fraction vapours, formed as a result of the distillation of the petroleum product, after preliminary removal of excess heat.

It is preferable to refresh the fluid working medium by adding thereto at least a portion of the liquid fraction from the column.

The invention also provides equipment for vacuum distillation of a petroleum product, comprising: a vacuum fractionating column with lines for feeding the petroleum product, discharging at least one liquid fraction, and discharging a residue; a reflux line for feeding part of the at least one liquid fraction to an upper portion of the column and a vapour-gaseous phase line which connects the upper portion of the column to the gas inlet of a liquid-gaseous ejector; characterised in that: the output of the liquid-gaseous ejector is connected with the inlet of a separator in which a gaseous phase and a liquid phase are separated, the gaseous phase and part of the liquid phase being discharged; a liquid output of the separator is connected with the inlet of a pump; the outlet of the pump is connected to the liquid inlet of the liquid-gaseous ejector; and the fluid working medium supplied to the liquid inlet of the liquid-gaseous ejector includes a condensate of petroleum fraction vapours, formed as a result of the distillation of the petroleum product, after preliminary removal of excess heat.

It is advisable to place a cooler between the liquid inlet of the ejector and the liquid outlet of the separator to remove excess heat from the liquid phase.

The connection of an additional separator inlet with the line for removing fluid fraction from the column provides refreshing of the fluid working medium with liquid fraction drawn from the column into the closed circuit of the vacuum-creating device.

The method for vacuum distillation of the petroleum product and the equipment provide effective removal of vapour-gaseous phase from the upper portion of the column followed with its condensation both in the flow part of the ejector and in the line after the ejector. The uncondensed gases (hydrocarbon gases) are simultaneously compressed to the pressure required for a user. The compression provides delivery from the separator of a hydrocarbon gas under pressure which may be used for technology requirements, and the excess of the liquid phase may be delivered as an intermediate product for further technological processing. In contrast to the prior art, the method does not include mixing of the top petroleum fractions with water and expulsion of condensate and hydrocarbon gases to the environment. Thus, the method is an ecologically pure one.

Moreover, the method makes it possible not to use a cooling condenser, for vapours of light petroleum fractions, in the line which connects the upper portion of the column with the vacuum-creating device, because condensation of the light petroleum fractions' vapours takes place in the ejector. The method also provides a reduction of the external circulation reflux rate of the column top, resulting in reduction of pressure difference there, and finally leads to a pressure reduction in the bottom of the column, this providing an increased yield of the low-boiling (light) products of vacuum distillation.

Such a method and construction of the equipment for vacuum distillation of a petroleum product provides a substantive decrease in environment contamination, decreased power consumption and, accordingly, an increase in the output of the equipment.

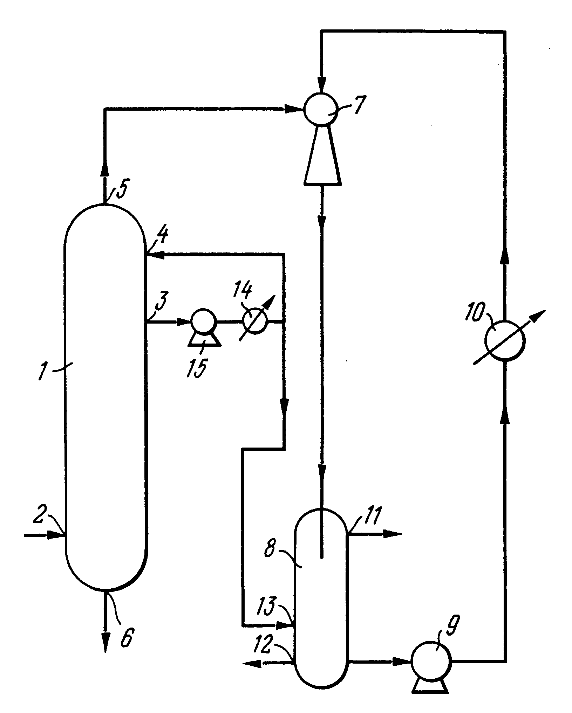

The invention is further described, by way of example, with reference to the accompanying drawing, schematically showing equipment for vacuum distillation of a petroleum product.

The equipment contains a rectifying (fractionating) vacuum column 1 equipped with a line 2 for feeding heated liquid product (petroleum stock), a liquid fraction output line 3, an external circulation reflux line 4, a vapour-gaseous phase output line 5, and a line 6 for output of the column bottoms. The line 5 connects the column top 1 with a liquid-gaseous ejector 7 which is connected in series with a separator 8 and a pump 9. The ejector 7, separator 8, and pump 9 constitute a vacuum-creating device, wherein the gas input of the ejector 7 is connected with the vapour-gaseous phase output line 5, the liquid input of the ejector 7 is connected with the output of the pump 9, the output of the ejector 7 is connected with the input of the separator 8, and the liquid output of the separator 8 is connected with the input of the pump 9.

A cooler 10 is mounted in the line between the liquid output of separator 8 and the liquid input of the ejector 7. The separator 8 is connected by a line 11 with the consumer's fuel system and by a line 12 with the petroleum product receiver (neither of which are shown). An additional input of the separator 8 is connected with the liquid fraction output line 3 of the column 1 by means of a line 13 for additional feeding of the vacuum-creating device with a liquid fraction from the column 1. A cooler 14 and a pump 15 are mounted in the line 3. The output of the cooler 14 is connected with the column 1 by the reflux line 4.

The vacuum distillation of a liquid product is carried out as follows.

The heated liquid product (petroleum stock) is fed to the column 1 under a pressure of 1.3 - 8.0 kPa (10 - 60 mm Hg) via the input line 2. The liquid fraction (vacuum gas oil) is discharged via the output line 3, passes through the pump 15, is cooled in the cooler 14, and is then divided into two flows, one of which flows via the line 4 is directed to the external circulating reflux of the upper column portion, and the other flow is directed via the line 13 through an additional input of the separator 8 of the vacuum-creating device. The liquid phase is gradually saturated with decomposition gases (hydrocarbon gases) evacuated from the upper column portion, this reducing vacuum extent in the column due to evolution of the said gases in the ejector nozzle. The latter causes energy consumption at the liquid phase pump of the vacuum-creating device. Therefore, in order to produce better vacuum, and to reduce power consumption in creating the latter, the liquid phase circulating in the vacuum-creating device is gradually refreshed using the liquid fraction coming from the column to the closed circuit of the vacuum-creating device.

From the top column portion, via the output line 5, the vapour-gaseous phase is discharged, being sucked off by the liquid-gas ejector 7 by means of the energy of liquid phase circulating in the closed circuit due to the pump 9. In the cooler 10 of the vacuum-creating device, excess heat is removed from the liquid phase, which excess is partially formed due to mechanical energy dissipation in the circuit of circulating liquid phase, and partially due to condensation and to cooling of the uncondensed gas which is sucked off from the column 1 by the liquid-gas ejector 7, thus providing temperature stabilization.

At the output of the liquid-gas ejector 7, due to energy transmission from the active liquid phase to the passive vapour-gaseous phase coming via the line 5 from the upper portion of the column 1, a two-phase mixture is formed having a pressure of greater than 0.11 MPa, which is fed to the separator 8. The final condensation of the vapour phase takes place in the mixture, which phase has not condensed in the flow-through part of the ejector 7. The mixture is divided in the separator 8 of the vacuum-creating device to form a gas phase and a liquid phase. The gas phase via the mains 11 is fed to the consumer's fuel system (not shown). The liquid phase, partially refreshed with the liquid fraction (vacuum gas oil) from the column 1, is directed to the pump 9 input. The liquid phase excess formed due to condensed vapours delivered to the ejector 7 from the upper portion of column 1, as well as due to the flow via the liquid fraction line 13, is delivered via the line 12 to the petroleum products receiver (not shown). During the start-up period of the vacuum-creating device the working medium used is any working fluid which is similar by its chemical and physical properties with the liquid phase, which phase is comprised of vapour condensate and the liquid fraction fed via the line 3. But gradually the initial working fluid is replaced by the mixture of vapour condensate and of the liquid fraction accumulated in the separator 8. The line 6 is used for discharge of the heavy fraction from vacuum distillation.

Thus, the suggested method and equipment for vacuum distillation of a product suggest the solution of actual problems in petroleum refining industry: the ecologically friendly technology of vacuum petroleum refining is realised, financial expenses are reduced for obtaining vacuum in a column, and the yield of light fractions is increased resulting from vacuum distillation of petroleum products.

## Claims

1. A method for vacuum distillation of a petroleum product, comprising:
feeding the petroleum product to a vacuum fractionating column (1) in which the petroleum product is separated into a vapour-gaseous phase containing vapours of petroleum fractions, at least one liquid fraction, and a residue;
feeding part of the at least one liquid fraction to an upper portion of the vacuum fractionating column (1); and
feeding the vapour-gaseous phase to a vacuum-creating device (7-9) which includes a liquid-gaseous ejector (7) having an inlet to which a fluid working medium is delivered;
characterised by:
providing condensation of the vapour-gaseous phase in the vacuum creating device (7-9) to produce a mixture which consists of a gaseous phase and a liquid phase comprising a condensate of petroleum fraction vapours;
separating the said mixture into the gaseous phase and the liquid phase and discharging the gaseous phase and part of the liquid phase from the vacuum-creating device (7-9); and
using as a part of the fluid working medium in the ejector (7) a condensate of petroleum fraction vapours, formed as a result of the distillation of the petroleum product, after preliminary removal of excess heat.

2. A method according to claim 1, in which part of the at least one liquid fraction is added to the fluid working medium.

3. Equipment for vacuum distillation of a petroleum product, comprising:
a vacuum fractionating column (1) with lines (2,3,6) for feeding the petroleum product, discharging at least one liquid fraction, and discharging a residue;
a reflux line (4) for feeding part of the at least one liquid fraction to an upper portion of the column (1); and
a vapour-gaseous phase line (5) which connects the upper portion of the column (1) to the gas inlet of a liquid-gaseous ejector (7);
characterised in that:
the output of the liquid-gaseous ejector (7) is connected with the inlet of a separator (8) in which a gaseous phase and a liquid phase are separated, the gaseous phase and part of the liquid phase being discharged;
a liquid output of the separator (8) is connected with the inlet of a pump (9);
the outlet of the pump (9) is connected to the liquid inlet of the liquid-gaseous ejector (7); and
the fluid working medium supplied to the liquid inlet of the liquid-gaseous ejector (7) includes a condensate of petroleum fraction vapours, formed as a result of the distillation of the petroleum product, after preliminary removal of excess heat.

4. Equipment according to claim 3, in which the liquid output of separator (8) is connected to the liquid inlet of the liquid-gaseous ejector (7) via a cooler (10).

5. Equipment according to claim 3 or 4, in which a liquid fraction output line (3) of the column (1) is connected to an additional inlet (13) of the separator (8).

6. Equipment according to claim 5, in which the said liquid fraction output line (3) is connected to the additional inlet (13) via a cooler (14).

## Patentansprüche

1. Verfahren zur Vakuumdestillation eines Erdölprodukts, mit den folgenden Schritten:
Einspeisen des Erdölprodukts in eine Vakuum-Fraktionierkolonne (1), in der das Erdölprodukt in eine Dampf/Gas-Phase getrennt wird, die Dämpfe von Erdölfraktionen, mindestens eine flüssige Fraktion und einen Rückstand enthält;
Einspeisen eines Teils der mindestens einen flüssigen Fraktion in einen oberen Abschnitt der Vakuum-Fraktionierkolonne (1); und
Einspeisen der Dampf/Gas-Phase in eine Vakuumerzeugungsvorrichtung (7-9), die einen Flüssigkeits-Gas-Saugstrahler (7) mit einem Einlaß aufweist, dem ein fluides Arbeitsmedium zugeführt wird;
gekennzeichnet durch:
Ausführung einer Kondensation der Dampf/Gas-Phase in der Vakuumerzeugungsvorrichtung (7-9) zur Herstellung eines Gemischs, das aus einer Gasphase und einer flüssigen Phase besteht, die ein Kondensat aus Erdöldampffraktionen aufweist;
Trennen des Gemischs in die Gasphase und die flüssige Phase und Austrag der Gasphase und eines Teils der flüssigen Phase aus der Vakuumerzeugungsvorrichtung (7-9); und
Verwendung eines Kondensats aus Erdöldampffraktionen, das als Ergebnis der Destillation des Erdölprodukts entsteht, nach vorherigem Entzug von überschüssiger Wärme als Teil des fluiden Arbeitsmediums im Saugstrahler (7).

2. Verfahren nach Anspruch 1, wobei ein Teil der mindestens einen flüssigen Fraktion dem fluiden Arbeitsmedium zugesetzt wird.

3. Anlage für die Vakuumdestillation eines Erdölprodukts, die aufweist:
eine Vakuum-Fraktionierkolonne (1) mit Leitungen (2, 3, 6) zum Einspeisen des Erdölprodukts, Ablassen mindestens einer flüssigen Fraktion und Ablassen eines Rückstands;
eine Rückflußleitung (4) zum Einspeisen eines Teils der mindestens einen flüssigen Fraktion in einen oberen Abschnitt der Kolonne (1); und
eine Dampf/Gas-Phasenleitung (5), die den oberen Abschnitt der Kolonne (1) mit dem Gaseinlaß eines Flüssigkeits-Gas-Saugstrahlers (7) verbindet;
dadurch gekennzeichnet, daß
der Auslaß des Flüssigkeits-Gas-Saugstrahlers (7) mit dem Einlaß eines Abscheiders (8) verbunden ist, in dem eine Gasphase und eine flüssige Phase getrennt werden, wobei die Gasphase und ein Teil der flüssigen Phase ausgetragen werden;
ein Flüssigkeitsauslaß des Abscheiders (8) mit dem Einlaß einer Pumpe (9) verbunden ist;
der Auslaß der Pumpe (9) mit dem Flüssigkeitseinlaß des Flüssigkeits-Gas-Saugstrahlers (7) verbunden ist; und
das dem Flüssigkeitseinlaß des Flüssigkeits-Gas-Saugstrahlers (7) zugeführte fluide Arbeitsmedium ein Kondensat aus Erdöldampffraktionen enthält, das als Ergebnis der Destillation des Erdölprodukts nach vorherigem Entzug von überschüssiger Wärme entsteht.

4. Anlage nach Anspruch 3, wobei der Flüssigkeitsauslaß des Abscheiden (8) über einen Kühler (10) mit dem Flüssigkeitseinlaß des Flüssigkeits-Gas-Saugstrahlers (7) verbunden ist.

5. Anlage nach Anspruch 3 oder 4, wobei eine Ausgangsleitung (3) für die flüssige Fraktion der Kolonne (1) mit einem zusätzlichen Einlaß (13) des Abscheiders (8) verbunden ist.

6. Anlage nach Anspruch 5, wobei die Ausgangsleitung (3) für die flüssige Fraktion über einen Kühler (14) mit dem zusätzlichen Einlaß (13) verbunden ist.

## Revendications

1. Procédé de distillation sous vide d'un produit pétrolier, comprenant:
le cheminement du produit pétrolier vers une colonne de fractionnement sous vide (1) dans laquelle le produit pétrolier est séparé en une phase vapeur/gaz contenant des vapeurs de fractions de pétrole, au moins une fraction de liquide et un résidu;
le cheminement d'une partie d'au moins la fraction de liquide vers une partie supérieure de la colonne de fractionnement sous vide (1); et
le cheminement de la phase vapeur/gaz vers un dispositif produisant un vide (7-9) qui inclut un éjecteur de liquide et de gaz (7) présentant une entrée vers laquelle un milieu de travail fluide est transmis;
caractérisé par:
la réalisation d'une condensation de la phase vapeur/gaz dans le dispositif produisant un vide (7-9) pour produire un mélange qui est constitué d'une phase gazeuse et d'une phase liquide comprenant un condensat de vapeurs de fractions de pétrole;
la séparation dudit mélange en la phase gazeuse et la phase liquide et le déchargement de la phase gazeuse et d'une partie de la phase liquide à partir du dispositif produisant un vide (7-9); et
l'utilisation en tant que partie du milieu de travail fluide dans l'éjecteur (7) du condensat de vapeurs de fractions de pétrole, formé par suite de la distillation du produit pétrolier, après un retrait préliminaire de l'excès de chaleur.

2. Procédé suivant la revendication 1, dans lequel une partie d'au moins la fraction de liquide est ajoutée au milieu de travail fluide.

3. Equipement destiné à une distillation sous vide d'un produit pétrolier, comprenant:
une colonne de fractionnement sous vide (1) dotée de conduits (2,3,6) servant à acheminer le produit pétrolier, à décharger au moins une fraction de liquide et à décharger un résidu;
un conduit de reflux (4) servant à acheminer une partie d'au moins la fraction de liquide vers une partie supérieure de la colonne (1); et
un conduit de phase vapeur/gaz (5) qui relie la partie supérieure de la colonne (1) à l'entrée de gaz d'un éjecteur de liquide et de gaz (7);
caractérisé en ce que:
la sortie de l'éjecteur de liquide et de gaz (7) est reliée à l'entrée d'un séparateur (8) dans lequel une phase gazeuse et une phase liquide sont séparées, la phase gazeuse et une partie de la phase liquide étant déchargées;
une sortie de liquide du séparateur (8) est reliée à l'entrée d'une pompe (9);
la sortie de la pompe (9) est reliée à l'entrée de liquide de l'éjecteur de liquide et de gaz (7); et
le milieu de travail fluide alimenté vers l'entrée de liquide de l'éjecteur de liquide et de gaz (7) inclut un condensat de vapeurs de fractions de pétrole, formé par suite de la distillation du produit pétrolier, après un retrait préliminaire de l'excès de chaleur.

4. Equipement suivant la revendication 3, dans lequel la sortie de liquide du séparateur (8) est reliée à l'entrée de liquide de l'éjecteur de liquide et de gaz (7) par l'intermédiaire d'un dispositif de refroidissement (10).

5. Equipement suivant la revendication 3 ou 4, dans lequel le conduit de sortie de fraction de liquide (3) de la colonne (1) est relié à une entrée supplémentaire (13) du séparateur (8).

6. Equipement suivant la revendication 5, dans lequel ledit conduit de sortie de fraction de liquide (3) est relié à l'entrée supplémentaire (13) par l'intermédiaire d'un dispositif de refroidissement (14).
